# EUROPEAN PATENT SPECIFICATION

(11) **EP 2 313 069 B1**
(45) Date of publication and mention of the grant of the patent: **28.09.2016**
(21) Application number: 09781935.3
(22) Date of filing: 18.08.2009
(51) Int. Cl.: A61K 8/29, A61K 8/35, A61Q 19/04

(54) **NOVEL COSMETIC OR DERMATOLOGICAL COMPOSITIONS**
NEUE KOSMETISCHE ODER DERMATOLOGISCHE ZUSAMMENSETZUNGEN
NOUVELLES COMPOSITIONS COSMÉTIQUES OU DERMATOLOGIQUES

(30) Priority: 19.08.2008 EP 08162601
(43) Date of publication of application: 27.04.2011
(73) Proprietor: DSM IP Assets B.V., 6411 TE Heerlen (NL)
(72) Inventor: BRAVE den, Kerstin, CH-4058 Basel (CH); MOSER, Heidi, CH-4416 Bubendorf (CH); WESTENFELDER, Horst, 67435 Neustadt a.d.W. (DE)
(74) Representative: Berg, Katja
(86) International application number: PCT/EP2009/060652
(87) International publication number: WO 2010/020626

(56) References cited:
- EP-A- 0 852 138
- EP-A- 1 911 437
- WO-A-03/090698
- WO-A-2005/004826
- WO-A-2007/101559
- WO-A-2009/074359
- DE-U1- 20 314 969
- US-A- 3 926 659
- US-A- 5 849 316
- 'New DSM Nutritional products enriches the PARSOL range with PARSOL TX', [Online] 16 May 2006, Retrieved from the Internet: <URL:http://www.newmaterials.com/News_Detai l_New_dsm_nutritional_products_enriches_the _parsol_range_with_parsoltx_4546.asp> [retrieved on 2012-08-30]

## Description

The present invention relates to novel cosmetic or dermatological compositions comprising at least one specific titanium dioxide and at least one self-tanning agent and a cosmetically acceptable carrier. The compositions are in particular suitable for artificial/sunless tanning and/or browning of human skin.

By the term "self-tanning agent" or "artificial/sunless tanning agent" are intended agents which, when topically applied onto the skin, in particular onto the face, elicit a tanning effect with an appearance more or less similar to that resulting from prolonged exposure to the sun (natural tanning) or under a UV lamp.

It is today important to look well and a tanned skin is always a sign of good health. However, natural tanning is not always desirable insofar as it requires prolonged exposure to UV radiation which causes browning of the skin but, on the other hand, induces skin damages such as increased wrinkling, elastosis, pigmentary changes, precancerous and cancerous skin lesions. Thus, it is desirable to have an alternative to natural tanning. Therefore, self-tanning ingredients are gaining more importance for various applications in the skin and sun care market. In addition to the classic self-tanners there are, for example, moisturizing preparations for face and body that gradually build up a very slight and discreet tan, after-sun preparations that prolong the tan, or sunscreens containing self-tanning ingredients.

The majority of cosmetic products for the artificial tanning of the skin are based on carbonyl derivatives which permit the formation of colored compounds by interaction with the amino acids of the skin. These compounds include mono- or polycarbonyl compounds, such as, for example, isatin, alloxan, ninhydrin, glyceraldehyde, mesotartaric aldehyde, glutaraldehyde, erythrulose and dihydroxyacetone (DHA).

Combinations of UV-filters with self-tanning ingredients are desirable products in today's cosmetic field. However, the combination of typical self-tanning ingredients such as erythrulose or dihydroxyacetone (DHA) with UV-filters, particularly with inorganic UV-filters, is still a difficult task due to the chemical properties of these substances. In particular the combination of inorganic pigments such as zinc oxide or titanium dioxide with self tanning ingredients such as erythrulose or DHA often leads to an unwanted brown discoloration of the composition. Also, there is an ongoing need for self-tanning products which act rapidly and impart a coloration similar to natural tanning.

WO2005004826 discloses the use of cosmetic and dermatological preparations, comprising one or more self-browning substances, for colouring the skin of multi-celled organisms, in particular, the skin of humans and animals, more particularly, for the colour matching of skin regions with different pigmentation.

It has now surprisingly been found, that the combination of certain titanium dioxides with a self tanning agent in cosmetic compositions overcomes the shortcomings of the prior art by significantly improving the stability of the compositions, reducing the discoloration of the formulations while showing an enhanced transparency, thus avoiding the so called 'whitening effect' on the skin. Furthermore, the colorations provided are more chromatic, more stable over time and have good homogeneity.

Thus, the invention relates to a cosmetic or dermatological composition comprising a self-tanning agent and at least one titanium dioxide which is substantially free of any aluminium coating characterized in that the self tanning agent is erythrulose and the titanium dioxide is a double coated titanium dioxide having an inner inorganic silica coating and an outer organic coating selected from silicone oils, alkyl silanes, olefinic acids, polyols or organophosphonic acids and mixtures thereof and has a primary particle size in the range of 2 to 100 nm.

The invention also relates to the topical application of compositions according to the invention for the coloring/browning of the skin to impart an appearance similar to natural tanning of the skin, for enhancement of the natural glow of the skin and/ or to provide a healthy appearance.

The present invention also relates to a method for artificially tanning or browning of the skin said method comprising the topical application of an effective amount of a composition according to the invention.

Furthermore, the invention relates to the use of at least one certain titanium dioxide for improving the stability and/ or for enhancing the coloring capability of a self-tanning agent.

The term 'effective amount' as used herein refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art.

The titanium dioxide used in the compositions according to the invention is substantially free of any aluminium coating as this leads to a more pronounced discoloration of the cosmetic or dermatological compositions compared to the use of titanium dioxide grades having no aluminium coating.

"Substantially free", as used herein, is understood to mean completely free of said coating, or inclusive of trace amounts of same. "Trace amounts" are those quantitative levels of a chemical constituent that are barely detectable and provide no benefit to the functional or aesthetic properties of the subject composition. Particularly, the titanium dioxide used according to the invention is completely free of any aluminium coating, meaning that the titanium dioxide has not been brought into contact with aluminium or an aluminium compound usually used for this kind of coating.

The titanium dioxide according to the invention is a double coated titanium dioxide having an inner inorganic silica coating and an outer organic coating (referred to as double coated titanium dioxide). Such coated titanium dioxides nanoparticles can be prepared according to the state of the art or are commercially available as PARSOL^{®} TX (INCI of ingredient: Titanium Dioxide, Silica, Dimethicone (C2H6OSi)xC4H12Si ex DSM Nutritional Products) or as UV-Titan X195 (coated with silica and treated with a silicone oil (i.e. methicone) ex Kemira).

The inner coating of the titanium dioxide particle with inorganic silica can be prepared according to the state of the art as e.g. described in EP-A 988 853, EP-A 1 284 277, EP0988853, and US 5562897, JP 2000319128.

The inner coating layer consists of minimum 0.5 wt%. Preferably 0.5-50 wt. % inorganic silica (based on titanium dioxide), most preferably of 1-20 wt.% The outer coating can be selected from the class of organic coatings such as organic polymers e.g. silicone oils (e.g. simethicones, methicones, dimethicones, polysilicone-15), alkyl silanes, olefinic acids such as stearic acid or polyols such as glycerol or organophosphonic acids. The outer coating layer consists of minimum 0.25 wt. % based on titanium dioxide. Preferably of 0.5-50 wt. %, most preferably of 0.5-10 wt.%.

Other usual organic coatings can additionally be present in order to yield multiple coated (such as e.g. triple coated) titanium dioxide. The other coatings can be applied before, after or together with the second outer coating. Other additional coatings which can be used comprise organic coatings such as stearic acid, silicones (silane derivatives such as triethoxycaprylylsilane or siloxane derivatives such as methicone, dimethicone, simethicone).

In a preferred embodiment, the titanium dioxide is a double coated titanium dioxide (having an inner inorganic silica coating) wherein the outer coating consists of simethicone, methicone, dimethicone (also known as polydimethylsiloxane), polysilicone-15, stearic acid, glycerol and mixtures thereof, in particular of methicone, dimethicone, stearic acid or mixtures thereof. Most preferably, the outer coating consists of methicone or dimethicone, in particular of dimethicone is. Most preferred according to the invention the titanium dioxide is UV-Titan X195 by Kemira and/or PARSOL^{®} TX by DSM Nutritional products which are titanium dioxide grades coated with silica (inner coating) and treated with a silicone oil such as in particular methicone (UV-Titan X195) or dimethicone (PARSOL^{®} TX) as outer coating. Most in particular PARSOL^{®} TX by DSM Nutritional products is used in the compositions according to the invention.

The primary particle size of the titanium dioxide is in the range from 2 to 100 nm, preferably in the range of 5 to 50 nm and the secondary particle size is preferably between 0.05 and 50 µm, preferably between 0.1 and 1 µm.

The crystalline form of the titanium dioxide may be of any crystal or amorphous type. For example, titanium dioxide may be any type of amorphous, rutil, anastase, brookite or a mixture thereof.

The titanium dioxide is generally present in the compositions according to the invention in proportions ranging from about 0.5 to about 50% by weight, preferably from about 1 to 25 wt.% in particular from about 1 to 10 wt.-%.

In a particular embodiment the ratio (w/w) of the self-tanning agent to the titanium dioxide is selected in the range of 0.1:1 to 1:0.1, such as in particular 0.5:1 to 1:0.5, most in particular in the range of 1:1.

Thus, the invention in particular relates to compositions comprising a double coated titanium dioxide having an inner silica coating and an outer coating selected from dimethicone or methicone, in particular dimethicone, and erythrulose as self tanning agent, wherein the ratio (w/w) of the self-tanning agent to the titanium dioxide is selected in the range of 0.5:1 to 1:0.5, such as in particular in the range of 1:1.

The self-tanning agent according to the present invention is erythrulose.

The self-tanning agent can be used in combination with at least one synthetic or natural direct dye and/or at least one indole derivative, such as those described in EP-425,324 and EP-456,545.

These self-tanning agent can also be used in combination with other synthetic or natural agents for coloring the skin.

By the term "agent for coloring the skin" is intended any compound having a specific affinity for the skin and which imparts thereto a lasting and noncovering (namely, having no tendency to opacify the skin) coloring, which is removed neither with water nor using a solvent, and which withstands both rubbing and washing with a solution comprising surfactants. Such a lasting coloring is therefore distinguished from the superficial and short-lived coloring contributed, for example, by a makeup pigment.

The additional coloring agents can also be selected, for example, from among plant extracts, such as, for example, extracts of "insoluble" redwoods of the Pterocarpus genus and of the Baphia genus, such as Pterocarpus santalinus, Pterocarpus osun, Pterocarpus soyauxii, Pterocarpus erinaceus, Pterocarpus indicus or Baphia nitida, such as those described in EP-971,683.

The coloring agents can also be iron oxide nanopigments for which the mean size of the individual particles is less than 100 nm, such as those described in EP-966,953.

The self-tanning agent is generally present in the compositions according to the invention in proportions ranging from 0.1% to 20% by weight with respect to the total weight of the composition and preferably from 0.2% to 8% by weight with respect to the total weight of the composition.

If erythrulose is used in combination with DHA, the cosmetic or dermatological composition preferably comprises about 1-5wt.-% erythrulose and about 1-15wt.-% DHA, advantageously about 1.5wt.-% erythrulose and 3.5wt.-% of DHA.

All percentages and ratios mentioned in this specification are by weight if nothing else is stated or evident.

The cosmetic or dermatological compositions according to the present invention can be prepared according to the state in the art. The compositions according to the invention additionally comprise a cosmetically or dermatologically acceptable carrier, vehicle or diluents.
Preferred are cosmetic or dermatological compositions for artificial/sunless tanning and/or browning of human skin. Preferably, the compositions which impart a self tanning effect to the skin are compositions for artificial/sunless tanning and/or browning, sunscreen compositions or moisturizers.

The self-tanning compositions in accordance with the invention can be provided in the form of creams, milks, gels, cream gels, oil-in-water emulsions, vesicular dispersions, fluid lotions, in particular vaporizable fluid lotions, or any other form generally used in cosmetics, in particular those usually suitable for self-tanning cosmetic compositions.

The compositions in accordance with the present invention can additionally comprise conventional cosmetic additives and adjuvants selected, in particular, from among fatty substances, organic solvents, ionic or nonionic thickeners, softeners, antioxidants, agents for combating free radicals, opacifiers, stabilizers, emollients, silicones, .alpha.-hydroxy acids, antifoaming agents, moisturizing agents, vitamins, insect repellents, substance P antagonists, anti-inflammatories, fragrances, preservatives, surfactants, fillers, polymers, propellants, basifying or acidifying agents, colorants or any other ingredient commonly used in the cosmetic and/or dermatological field, in particular for the production of self-tanning compositions in the form of emulsions.
The fatty substances can be an oil or a wax, or mixture thereof. By the term "oil" is intended a compound which is liquid at ambient temperature. By the term "wax" is intended a compound which is solid or substantially solid at ambient temperature and for which the melting point is generally greater than 35.degree. C.
Exemplary oils are mineral oils (liquid paraffin); vegetable oils (sweet almond, macadamia, blackcurrant seed or jojoba oil); synthetic oils, such as perhydrosqualene, fatty alcohols, acids or esters (such as the C.sub.12 -C.sub.15 alkyl benzoate marketed under the trademark "Finsolv TN" by Finetex, octyl palmitate, isopropyl lanolate or triglycerides, including those of capric/caprylic acids), or oxyethylenated or oxypropylenated fatty esters and ethers; silicone oils (cyclomethicone, polydimethylsiloxanes or PDMS); fluorinated oils; polyalkylenes and their mixtures.
Exemplary waxy compounds are paraffin wax, carnauba wax, beeswax or hydrogenated castor oil.
And exemplary organic solvents include the lower alcohols and polyols having at most 8 carbon atoms.
The thickeners are advantageously selected, in particular, from among the crosslinked polyacrylic acids or modified or unmodified guar gums and celluloses, such as hydroxypropylated guar gum, methylhydroxyethylcellulose and hydroxypropylmethylcellulose.

The compositions according to this invention can additionally comprise further organic or inorganic light screening agents which are active in the UV-A and/or UV-B regions (absorbers), such light screening agents being water-soluble, fat-soluble or insoluble in commonly used cosmetic solvents.

Examples of UV-B or broad spectrum screening agents, i.e. substances having absorption maximums between about 290 nm and 340 nm may be organic or inorganic compounds. Organic UV-B or broadband screening agents are e.g. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as PARSOL^{®} SLX; drometrizole trisiloxane (Mexoryl XL); imidazole derivatives such as e.g. 2-phenyl benzimidazole sulfonic acid and its salts (PARSOL^{®}HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL^{®} EHS, Neo Heliopan OS), isooctyl salicylate or homomenthyl salicylate (homosalate, PARSOL^{®} HMS, Neo Heliopan HMS) and the like; triazine derivatives such as ethylhexyl triazone (Uvinul T-150), diethylhexyl butamido triazone (Uvasorb HEB) and the like. Encapsulated UV-filters such as encapsulated ethylhexyl methoxycinnamate (Eusolex UV-pearls) or microcapsules loaded with UV-filters as e.g. dislosed in EP 1471995 and the like; Examples of broad spectrum or UV A screening agents i.e. substances having absorption maximums between about 320 nm and 400 nm may be organic or inorganic compounds e.g. dibenzoylmethane derivatives such as 4-tert.-butyl-4'-methoxydibenzoyl-methane (PARSOL^{®} 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (Tinosorb M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-Diethylamino-2-hydroxy-benzoyl)-benzoic acid hexylester (Uvinul A plus) as described in the European Patent Publication EP 1046391; Ionic UV-A filters as described in the International Patent Publication WO2005080341 A1; Pigments such as microparticulated ZnO and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.
As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

The light screening agents are generally present in the compositions according to the invention in proportions ranging from 0.1 % to 20% by weight with respect to the total weight of the composition and preferably ranging from 0.2% to 15% by weight with respect to the total weight.

Of course, one skilled in this art will take care to select the abovementioned optional additional compound or compounds and/or their amounts such that the advantageous properties intrinsically associated with the combination in accordance with the invention are not, or not substantially, detrimentally affected by the envisaged addition or additions.
The compositions according to the invention can be formulated according to techniques well known to this art, in particular those suited for the preparation of emulsions of oil-in-water or water-in-oil type.

These compositions can be provided, in particular, in the form of a simple or complex emulsion (O/W, W/O, O/W/O or W/O/W), such as a cream or a milk, or in the form of a gel or of a cream gel, or in the form of a lotion, of a powder or of a solid tube and can optionally be packaged as an aerosol and provided in the form of a foam or spray.
The compositions according to the invention are preferably formulated an oil-in-water or water-in-oil emulsion.

The cosmetic and/ or dermatological compositions according to the invention have a pH in the range of 3-10, preferably in the range of pH of 4-8, most preferred in the range of pH 4-6.

The invention also relates to a method for artificially tanning and/or browning the skin, which comprises topically applying an effective amount of a cosmetic composition as described above on the skin for such a period of time as is required to elicit the desired artificial/sunless tanning effect.

The invention also relates to the use of a titanium dioxide as described above for the purpose of improving the stability of a self-tanning agent in cosmetic or dermatological compositions and/ or for reducing the discoloration of cosmetic or dermatological compositions comprising of a self-tanning agent.

In a preferred embodiment the invention relates to cosmetic or dermatological compositions comprising additionally a hydrocolloid selected from cellulose gum, xanthan gum, PVM/MA decadiene crosspolymer or hydroxyethyl cellulose.

The cosmetic and/ or dermatological compositions according to the invention are preferably applied at least once per day but can also be applied several times a day e.g. two or three times a day.

The amount of the cosmetic and/or dermatological composition which is to be applied to the skin depends on the concentration of the active ingredients in the compositions and the desired cosmetic or pharmaceutical effect. For example, application can be such that a crème is applied to the skin. A cream is usually applied in an amount of about 1 to 2 mg crème/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

In order to further illustrate the present invention and the advantages thereof, the following specific example is given, it being understood that same is intended only as illustrative and in nowise limitative.

### EXAMPLE 1

The following basic formulation was prepared

| **Phase** | **Ingredients** | **NCI Name** | **% w/w** |
|---|---|---|---|
| | | | |
| A | Estol 3650 | Glyceryl Myristate | 3.50 |
| | Lanette 16 | Cetyl Alcohol | 3.00 |
| | Brij 72 | Steareth-2 | 2.00 |
| | Brij 721 | Steareth-21 | 2.00 |
| | Butylated Hydroxytoluene | BHT | 0.05 |
| | Phenonip | Phenoxyethanol & Methylparaben & Ethylparaben & Butylparaben & Propylparaben & Isobutylparaben | 0.80 |
| | Dow Corning 200/100 cs | Dimethicone | 1.00 |
| | Finsolv TN | C12-15 Alkyl Benzoate | 16.00 |
| | **Titanium dioxide** | | **5.00%** |
| | | | |
| B | Keltrol CG-T | Xanthan Gum | 0.30 |
| | | | |
| C | 1,2-Propanediol | Propylene Glycol | 3.00 |
| | Edeta BD | Disodium EDTA | 0.10 |
| | Water dem. | Aqua | add 100 |
| | | | |
| **D** | **self tanning agent** | | **5.00%** |
| | | | |
| E | Neutralizing agent | - | q.s. |

### Procedure

1 Heat part A to 80°C while stirring. The different TiO₂ were added in the hot oil phase under agitation.
2 When everything is homogenous add part B under agitation
3 Heat part C to 80°C and add to the oil phase while stirring and homogenizing the emulsion.
4 Erythrulose and/or DHA (part D) were added to the final formulation under agitation at 30°C.
5 Cool down to RT and adjust the pH to 5 with part E if necessary

The formulations were stored at room temperature and 43°C. From each sample the colour change was measured with Minolta Spectrophotometer CM-3600d (Lab-value (L*a*b)) after preparation and after two weeks storage time. The average of three measurements was taken for the calculation of the degree of discoloration (ΔE).

ΔE of formulations containing Dihydroxyaceton (5%) as self tanning agent and different TiO₂ grades:

| **Titanium Dioxide** | **ΔE RT** | **ΔE 43°C** |
|---|---|---|
| none | 0.28 | 0.87 |
| PARSOL^{®} TX ex DSM Nutritional Products Ltd. Double coated titanium dioxide with silica & polydimethylsiloxane | 0.43 | 5.80 |
| Uvinul^{®} TiO₂ ex BASF Single coated titanium dioxide with Trimethoxycaprylsilane | 2.49 | 6.31 |
| AEROXIDE^{®} TiO₂ P 25 ex Degussa non coated titanium dioxide | 1.71 | 8.75 |
| Tayca MT 100T ex Tayca Corporation Double coated titanium dioxide with Alumina & Stearic Acid | 11.03 | 26.58 |
| Eusolex^{®}T-2000 ex Merck Double coated titanium dioxide with Alumina & Simethicone | 5.00 | 19.38 |
| T-Lite SF ex BASF Double coated titanium dioxide with Aluminium Hydroxide & Dimethicone/ Methicone Copolymer | 5.39 | 14.55 |

ΔE of formulations containing Erythrulose (5%) as self tanning agent and different TiO₂ grades:

| **Titanium Dioxide** | **ΔE RT** | **ΔE 43°C** |
|---|---|---|
| none | 1.03 | 5.50 |
| PARSOL^{®} TX ex DSM Nutritional Products Ltd. Double coated titanium dioxide with silica & polydimethylsiloxane | 1.66 | 6.68 |
| Uvinul^{®} TiO₂ ex BASF Single coated titanium dioxide with Trimethoxycaprylsilane | 3.39 | 13.86 |
| AEROXIDE^{®} TiO₂ P 25 ex Degussa non coated titanium dioxide | 8.30 | 12.38 |
| Tayca MT 100T ex Tayca Corporation Double coated titanium dioxide with Alumina & Stearic Acid | 5.43 | 13.16 |
| Eusolex^{®}T-2000 ex Merck Double coated titanium dioxide with Alumina & Simethicone | 5.83 | 11.82 |
| T-Lite SF ex BASF Double coated titanium dioxide with Aluminium Hydroxide & Dimethicone/ Methicone Copolymer | 6.66 | 14.87 |

ΔE of formulations containing a combination of Dihydroxyaceton (2.5%) and Erythrulose (2.5%) as self tanning agent and different TiO₂ grades:

| **Titanium Dioxide** | **ΔE RT** | **ΔE 43°C** |
|---|---|---|
| none | 0.37 | 4.51 |
| PARSOL^{®} TX ex DSM Nutritional Products Ltd. Double coated titanium dioxide with silica & dimethicone | 1.3 | 6.18 |
| Uvinul^{®} TiO₂ ex BASF Single coated titanium dioxide with Trimethoxycaprylsilane | 6.16 | 13.24 |
| AEROXIDE^{®} TiO2 P 25 ex Degussa non coated titanium dioxide | 6.76 | 10.86 |
| Tayca MT 100T ex Tayca Corporation Double coated titanium dioxide with Alumina & Stearic Acid | 7.52 | 21.12 |

As can be retrieved from the results, the aluminium coated TiO₂ grades discolour more than TiO₂ grades containing no aluminium coating. The best results are obtained with the double coated titanium PARSOL^{®} TX (i.e. a titanium dioxide coated with silica (= inner coating) and treated with a silicone oil (such as dimethicone, = outer coating))

### EXAMPLE 2

| *Phase* | *Ingredients* | *INCI Name* | *Wt.-%* |
|---|---|---|---|
| | | | |
| A | Cremophor A6 | Ceteareth 6, Stearylalcohol | 2.50 |
| | Cremophor A25 | Ceteareth 25 | 2.50 |
| | Cetylalcohol | Cetyl Alcohol | 5.00 |
| | Stearic Acid | Stearic Acid | 3.00 |
| | Paraffin oil | Mineral Oil | 5.50 |
| | Tegosoft M | Isopropyl myristate | 5.50 |
| | Titanium Dioxide | Parsol TX by DSM | 5.00 |
| | | | |
| B | Water | Aqua | 60.50 |
| | Glycerin | Glycerin | 5.00 |
| | Phenonip | Phenoxyethanol (and) Methylparaben | 0.50 |
| | | (and) Ethylparaben (and) Butylparaben | |
| | | (and) Propylparaben | |
| | | | |
| | ERYTRULOSE | ERYTHRULOSE | 5.00 |
| | Water | Aqua | Ad 100 |
| | | | |
| | | Total | *100.00* |

The ingredients A are added together and heated to 70°. The ingredients B are added together and heated to 70°. The oil phase A is added to the water phase B while homogenizing. Afterwards the erythrulose is added during stirring. The pH is adjusted with aqueous NaOH to about 4.7. The formulation does not discolor during storage which can be assessed by Lab-value (L*a*b) or visually at different storage conditions and over different periods of time.

## Claims

1. A cosmetic or dermatological composition comprising a self-tanning agent and at least one titanium dioxide which is substantially free of any aluminium coating **characterized in that** the self tanning agent is erythrulose and the titanium dioxide is a double coated titanium dioxide having an inner inorganic silica coating and an outer organic coating selected from silicone oils, alkyl silanes, olefinic acids, polyols or organophosphonic acids and mixtures thereof and has a primary particle size in the range of 2 to 100 nm.

2. A cosmetic or dermatological composition according to claim 1 wherein the outer coating is selected from simethicone, methicone, dimethicone, polysilicone-15, stearic acid, glycerol and mixtures thereof.

3. A cosmetic or dermatological composition according to claim 1 wherein the outer coating is selected from methicone, dimethicone, polysilicone-15 or stearic acid.

4. A cosmetic or dermatological composition according to claim 1 wherein the outer coating is selected from methicone or dimethicone.

5. A cosmetic or dermatological composition according to anyone of claims 1 to 4 wherein the total amount of the erythrulose is in the range from about 0.1% to 10% wt.-% based on the total weight of the composition.

6. A cosmetic or dermatological composition according to anyone of claims 1 to 5 wherein the titanium dioxide is present in an amount from 1 to 25 wt.-% based on the total weight of the compositions.

7. A cosmetic or dermatological composition according to any one of claims 1 to 6 further comprising at least one further light screening agent.

8. A cosmetic or dermatological composition according to any one of claims 1 to 7, **characterized in that** the primary particle size of titanium dioxide is selected in the range of 5 to 50 nm.

9. Use of a composition as in any one of claims 1 to 8 for artificial/sunless tanning and/or browning of human skin.

10. A cosmetic or dermatological composition according to anyone of claims 1 to 8 for the protection of human skin against UV-radiation.

11. A cosmetic method for artificial tanning or browning of human skin, said method comprising topically applying an effective amount of a cosmetic composition as defined in anyone of claims 1 to 8 to the skin.

## Patentansprüche

1. Kosmetische oder dermatologische Zusammensetzung, umfassend ein Selbstbräunungsmittel und wenigstens ein Titandioxid, das im Wesentlichen frei von jeglicher Aluminiumbeschichtung ist, **dadurch gekennzeichnet, dass** das Selbstbräunungsmittel Erythrulose ist und das Titandioxid ein doppelt beschichtetes Titandioxid mit einer inneren anorganischen Siliciumdioxid-Beschichtung und einer äußeren organischen Beschichtung ausgewählt aus Siliconölen, Alkylsilanen, olefinischen Säuren, Polyolen und Organophosphonsäuren und Gemischen davon ist und eine Primärpartikelgröße in dem Bereich von 2 bis 100 nm aufweist.

2. Kosmetische oder dermatologische Zusammensetzung gemäß Anspruch 1, wobei die äußere Beschichtung ausgewählt ist aus Simethicon, Methicon, Dimethicon, Polysilicon-15, Stearinsäure, Glycerol und Gemischen davon.

3. Kosmetische oder dermatologische Zusammensetzung gemäß Anspruch 1, wobei die äußere Beschichtung ausgewählt ist aus Methicon, Dimethicon, Polysilicon-15 und Stearinsäure.

4. Kosmetische oder dermatologische Zusammensetzung gemäß Anspruch 1, wobei die äußere Beschichtung ausgewählt ist aus Methicon und Dimethicon.

5. Kosmetische oder dermatologische Zusammensetzung gemäß einem der Ansprüche 1 bis 4, wobei die Gesamtmenge der Erythrulose in dem Bereich von etwa 0,1 % bis 10 Gew.-% bezogen auf das Gesamtgewicht der Zusammensetzung beträgt.

6. Kosmetische oder dermatologische Zusammensetzung gemäß einem der Ansprüche 1 bis 5, wobei das Titandioxid in einer Menge von 1 bis 25 Gew.-% bezogen auf das Gesamtgewicht der Zusammensetzung vorhanden ist.

7. Kosmetische oder dermatologische Zusammensetzung gemäß einem der Ansprüche 1 bis 6, ferner umfassend wenigstens ein weiteres Sonnenschutzmittel.

8. Kosmetische oder dermatologische Zusammensetzung gemäß einem der Ansprüche 1 bis 7, **dadurch gekennzeichnet, dass** die Primärpartikelgröße von Titandioxid in dem Bereich von 5 bis 50 nm ausgewählt ist.

9. Verwendung einer Zusammensetzung gemäß einem der Ansprüche 1 bis 8 zum künstlichen/sonnenlosen Bräunen und/oder Bräunen von menschlicher Haut.

10. Kosmetische oder dermatologische Zusammensetzung gemäß einem der Ansprüche 1 bis 8 für den Schutz von menschlicher Haut gegen UV-Strahlung.

11. Kosmetisches Verfahren zum künstlichen Bräunen oder Bräunen von menschlicher Haut, wobei das Verfahren das topische Aufbringen einer wirksamen Menge einer kosmetischen Zusammensetzung gemäß einem der Ansprüche 1 bis 8 auf die Haut umfasst.

## Revendications

1. Composition cosmétique ou dermatologique, comprenant un agent auto-bronzant et au moins un dioxyde de titane qui est sensiblement dépourvu de tout revêtement en aluminium, **caractérisée en ce que** l'agent auto-bronzant est l'érythrulose et le dioxyde de titane est un dioxyde de titane à double revêtement ayant un revêtement en silice inorganique interne et un revêtement organique externe choisi parmi les huiles de silicone, les alkylsilanes, les acides oléfiniques, les polyols ou les acides organophosphoniques, et des mélanges de ceux-ci, et possède une taille de particule primaire dans la plage allant de 2 à 100 nm.

2. Composition cosmétique ou dermatologique selon la revendication 1, dans laquelle le revêtement externe est choisi parmi la siméthicone, la méthicone, la diméthicone, la polysilicone-15, l'acide stéarique, le glycérol, et des mélanges de ceux-ci.

3. Composition cosmétique ou dermatologique selon la revendication 1, dans laquelle le revêtement externe est choisi parmi la méthicone, la diméthicone, la polysilicone-15 ou l'acide stéarique.

4. Composition cosmétique ou dermatologique selon la revendication 1, dans laquelle le revêtement externe est choisi parmi la méthicone ou la diméthicone.

5. Composition cosmétique ou dermatologique selon l'une quelconque des revendications 1 à 4, dans laquelle la quantité totale d'érythrulose se trouve dans la plage allant d'environ 0,1% à 10% en poids, sur la base du poids total de la composition.

6. Composition cosmétique ou dermatologique selon l'une quelconque des revendications 1 à 5, dans laquelle le dioxyde de titane est présent selon une quantité allant de 1 à 25% en poids, sur la base du poids total de la composition.

7. Composition cosmétique ou dermatologique selon l'une quelconque des revendications 1 à 6, comprenant en outre au moins un autre agent photo-protecteur.

8. Composition cosmétique ou dermatologique selon l'une quelconque des revendications 1 à 7, **caractérisée en ce que** la taille de particule primaire de dioxyde de titane est choisie dans la plage allant de 5 à 50 nm.

9. Utilisation d'une composition selon l'une quelconque des revendications 1 à 8, pour le bronzage artificiel/sans soleil et/ou le brunissement de la peau humaine.

10. Composition cosmétique ou dermatologique selon l'une quelconque des revendications 1 à 8, pour la protection de la peau humaine contre le rayonnement UV.

11. Méthode cosmétique de bronzage artificiel ou de brunissement de la peau humaine, ladite méthode comprenant l'application topique à la peau d'une quantité efficace d'une composition cosmétique telle que définie selon l'une quelconque des revendications 1 à 8.
